# EUROPEAN PATENT SPECIFICATION

(11) **EP 3 928 785 B1**
(45) Date of publication and mention of the grant of the patent: **31.07.2024**
(21) Application number: 20924983.8
(22) Date of filing: 09.05.2020
(51) Int. Cl.: A61K 38/47, A61K 9/00, A61K 31/704, A61K 45/06, A61P 31/14, C12N 9/36, A61P 31/12, A23L 33/10, A23L 33/17

(54) **MEDICAMENT AND FOOD FOR USE IN PREVENTING OR TREATING NOVEL CORONAVIRUS PNEUMONIA COVID-19**
ARZNEIMITTEL UND NAHRUNGSMITTEL ZUR VERWENDUNG ZUR VORBEUGUNG ODER BEHANDLUNG DER DURCH DAS NEUARTIGE CORONAVIRUS VERURSACHTEN PNEUMONIE COVID-19
MÉDICAMENT ET ALIMENT POUR LEUR UTILISATION DANS LA PRÉVENTION OU LE TRAITEMENT DE LA NOUVELLE PNEUMONIE DU CORONAVIRUS COVID-19

(30) Priority: 27.04.2020 CN 202010341811
(43) Date of publication of application: 29.12.2021
(73) Proprietor: Guangzhou Century Clinical Research Co., Ltd, Guangzhou, Guangdong 510530 (CN); Guangzhou Xin-Chuangyi Biopharmaceutical Co., Ltd, Huangpu District Guangzhou, Guangdong 510535 (CN); Guangzhou Welman New Drug R&D Co,. Ltd., Guangzhou, Guangdong 510620 (CN); Xiangbei Welman Pharmaceutical Co., Ltd, Hunan 410331 (CN); Nanjing Kangfushun Pharmaceutical Co., Ltd, Nanjing, Jiangsu 210046 (CN)
(72) Inventor: SUN, Mingjie, Guangzhou Guangdong 510620 (CN); SUN, Tianyu, Guangzhou Guangdong 510620 (CN); LI, Changqing, Guangzhou Guangdong 510620 (CN)
(74) Representative: Kraus & Lederer PartGmbB
(86) International application number: PCT/CN2020/089422
(87) International publication number: WO 2021/217702

(56) References cited:
- EP-A1- 1 757 303
- EP-A1- 4 104 849
- WO-A1-2019/046664
- WO-A1-2020/240472
- CN-A- 1 255 859
- CN-A- 1 449 822
- CN-A- 1 548 152
- CN-A- 1 579 189
- CN-A- 1 618 463
- US-B1- 6 329 339
- LUO PAN ET AL: "Pharmacological perspective: glycyrrhizin may be an efficacious therapeutic agent for COVID-19", INTERNATIONAL JOURNAL OF ANTIMICROBIAL AGENTS, ELSEVIER, AMSTERDAM, NL, vol. 55, no. 6, 24 April 2020 (2020-04-24), XP086159031, ISSN: 0924-8579, [retrieved on 20200424], DOI: 10.1016/J.IJANTIMICAG.2020.105995
- SARAHS CHERIAN ET AL: "Perspectives for repurposing drugs for the coronavirus disease 2019", INDIAN JOURNAL OF MEDICAL RESEARCH, vol. 151, no. 0, 1 February 2020 (2020-02-01), IN, pages 160 - 171, XP055727615, ISSN: 0971-5916, DOI: 10.4103/ijmr.IJMR_585_20
- ZHOU WAIMIN; ZHAO FUMING; LI BANG; LONG TIAN: "The Clinical Value of Glycyrrhizinate Diamine in the Treatment of Patients with Common New Coronavirus Pneumonia", CHINESE JOURNAL OF VIROLOGY, vol. 36, no. 2, 31 March 2020 (2020-03-31), pages 160 - 164, XP055862780, ISSN: 1000-8721, DOI: 10.13242/j.cnki.bingduxuebao.003679

## Description

### TECHNICAL FIELD

The present invention relates to the field of medicine, and in particular, to a medicament or combination comprising lysozyme and glycyrrhizic acid or a salt thereof for use in preventing or treating the coronavirus pneumonia COVID-19.

### BACKGROUND ART

Some medical institutions in Wuhan, China, received patients with a pneumonia of unknown cause in December, 2019, which was identified as a pneumonia caused by a 2019 novel coronavirus (coronavirus pneumonia COVID-19). Subsequently, an epidemic began to spread, and the coronavirus pneumonia COVID-19 outbreak occurred in Wuhan, China, and became a global pandemic. The 2019 novel coronavirus is highly contagious, and the mortality rate of patients with severe coronavirus pneumonia COVID-19 is high. At present, there have been cumulatively no less than 2.8 million patients with confirmed infections globally, and the cumulative death toll is no less than 200,000. The disease has caused an unprecedented huge loss of lives and properties to the human society.

COVID-19 is a new virus belonging to the family of coronaviridae. COVID-19 has certain similarities to SARS coronavirus, but also has obvious differences, especially in terms of viral gene sequence, asymptomatic carriers, infectivity, clinical symptoms, histopathology etc. Pathological observations have shown that the degree of pulmonary fibrosis in patients with the coronavirus pneumonia COVID-19 is less than that in patients with the atypical pneumonia SARS; however, pulmonary hyperplasia and obstruction in the patients with the coronavirus pneumonia COVID-19 are severer than those in the patients with the atypical pneumonia SARS.

Currently, some drugs which may be used for treating the coronavirus pneumonia COVID-19 have been recommended all over the world. However, many of them are intended for emergency use or compassionate use, and there are still many shortcomings. For example, broad-spectrum antiviral drugs have no obvious activity against COVID-19, and many drugs for the symptomatic treatment of the viral pneumonia also have poor effects. There are also a large number of drugs which may inhibit the virus, but have obvious toxicity. Therefore, there is an urgent need for a safe and effective medicament for treating the coronavirus pneumonia COVID-19.

SARAHS CHERIAN ET AL("Perspectives for repurposing drugs for the coronavirus disease 2019", INDIAN JOURNAL OF MEDICAL RESEARCH, vol.151,no.0,1 February 2020 (2020-02-01), pages160-171, XP055727615, IN ISSN:0971-5916,DOI:10.4103/ijmr.lJMR585_20.) reviews current therapies for COVID-19 and in particular drug repurposing as an effective drug discovery approach from earlier approved drugs.

### SUMMARY OF THE INVENTION

The invention is set out in the appended set of claims.

A first objective of the present invention is to provide a medicament for use in preventing or treating coronavirus pneumonia COVID-19. In order to achieve the objective, the technical solution used in the present invention is:
a medicament for use in preventing or treating the coronavirus pneumonia COVID-19, wherein the medicament comprises lysozyme and glycyrrhizic acid or a salt thereof.

In some embodiments, the medicament further comprises an additional ingredient useful for preventing or treating the coronavirus pneumonia COVID-19.

In some embodiments, the additional ingredient useful for preventing or treating the coronavirus pneumonia COVID-19 is one or more selected from an interferon, oseltamivir or a salt thereof, lopinavir, ritonavir, favipiravir, ribavirin, remdesivir, chloroquine or a salt thereof, hydroxychloroquine or a salt thereof, arbidol or a salt thereof, an interleukin inhibitor, a tumor necrosis factor inhibitor, a janus kinase inhibitor, a glucocorticoid, a protease inhibitor, and an intestinal microecological regulator.

In some embodiments, the protease inhibitor may be selected from ulinastatin, sivelestat, nafamostat, or tranexamic acid.

In some embodiments, the interleukin inhibitor may be selected from tocilizumab.

In some embodiments, the tumor necrosis factor inhibitor may be selected from adalimumab, infliximab, or etanercept.

In some embodiments, the janus kinase inhibitor may be selected from tofacitinib or baricitinib.

In some embodiments, the intestinal microecological regulator may be selected from prebiotics or probiotics.

In some embodiments, the dosage form of the medicament is selected from various dosage forms suitable for medicinal use, such as an oral dosage form, an injection dosage form, and an inhalation dosage form.

In some embodiments, the medicament is presented as preparations with various release forms, such as a normal release preparation, a delayed release preparation, a sustained release preparation, or a controlled release preparation.

In some embodiments, the medicament further comprises a pharmaceutically applicable excipient.

In some embodiments, the weight ratio of the lysozyme to the glycyrrhizic acid or the salt thereof in the medicament is 100 : 1 to 10 : 1. In some embodiments, the lysozyme and the glycyrrhizic acid or the salt thereof have a significant synergistic effect.

In some embodiments, the daily dosage of the lysozyme may be 0.5 g to 20 g.

In some embodiments, the lysozyme may be administered during the non-sleeping time at a dosage frequency of once every 1 to 24 hours, such as once every 1 hour, every 2 hours, every 4 hours, every 6 hours, every 8 hours, every 12 hours, or every 24 hours.

A second objective of the present invention is to provide a combination for use in preventing or treating the coronavirus pneumonia COVID-19. In order to achieve the objective, the technical solution used in the present invention is:
a combination for use in preventing or treating the coronavirus pneumonia COVID-19, wherein the combination comprises an effective amount of lysozyme and glycyrrhizic acid or a salt thereof.

In some embodiments, the combination further comprises an additional ingredient useful for preventing or treating the coronavirus pneumonia COVID-19.

In some embodiments, the additional ingredient useful for preventing or treating the coronavirus pneumonia COVID-19 is one or more selected from an interferon, oseltamivir or a salt thereof, lopinavir, ritonavir, favipiravir, ribavirin, remdesivir, chloroquine or a salt thereof, hydroxychloroquine or a salt thereof, arbidol or a salt thereof, an interleukin inhibitor, a tumor necrosis factor inhibitor, a janus kinase inhibitor, a glucocorticoid, a protease inhibitor, and an intestinal microecological regulator.

In some embodiments, the lysozyme and the glycyrrhizic acid or the salt thereof in the combination are combined in various manners. In some embodiments, the lysozyme and the glycyrrhizic acid or the salt thereof exist separately in different pharmaceutical products, for example, applied in a combined packaging or combined medication manner. In other embodiments, the lysozyme and the glycyrrhizic acid or the salt thereof coexist in the same pharmaceutical product, for example, administered as a compound medicament.

In some embodiments, the lysozyme and the additional ingredient useful for preventing or treating the coronavirus pneumonia COVID-19 in the combination may be combined in various manners. In some embodiments, the lysozyme and the additional ingredient exist separately in different pharmaceutical products, for example, applied in a combined packaging or combined medication manner. In other embodiments, the lysozyme and the additional ingredient coexist in the same pharmaceutical product, for example, administered as a compound medicament.

In some embodiments, for the administration of the combination, there are a variety of methods, such as oral administration, injection, and inhalation.

In some embodiments, the combination is presented as preparations with various release forms, such as a normal release preparation, a delayed release preparation, a sustained release preparation, and a controlled release preparation.

In some embodiments, the combination is further comprise a pharmaceutically applicable excipient.

In some embodiments, the weight ratio of the lysozyme to the glycyrrhizic acid or the salt thereof in the combination is 100 : 1 to 10 : 1. In some embodiments, the lysozyme and the glycyrrhizic acid or the salt thereof have a significant synergistic effect.

In some embodiments, the daily dosage of the lysozyme is 0.5 g to 20 g.

In some embodiments, the combination is administered during the non-sleeping time at a dosage frequency of once every 1 to 24 hours, such as once every 1 hour, every 2 hours, every 4 hours, every 6 hours, every 8 hours, every 12 hours, or every 24 hours.

In some embodiments, the subject is tested positive for COVID-19 coronavirus nucleic acid.

In some embodiments, the subject is clinically diagnosed with the coronavirus pneumonia COVID-19.

In some embodiments, in addition to being tested positive for COVID-19 coronavirus nucleic acid, or being clinically diagnosed with the coronavirus pneumonia COVID-19, the subject further suffers from increased airway resistance or decreased expiratory volume.

In some embodiments, in addition to being tested positive for the COVID-19 coronavirus nucleic acid, or being clinically diagnosed with the coronavirus pneumonia COVID-19, the subject further suffers from impaired intestinal barrier function.

In some embodiments, the combination comprising lysozyme is prepared into a food.In some embodiments, the lysozyme and the glycyrrhizic acid or the salt thereof in the combination are combined in various manners. In some embodiments, the lysozyme and the glycyrrhizic acid or the salt thereof exist separately in different food products, for example, combined in a combined packaging or combined medication manner. In other embodiments, the lysozyme and the glycyrrhizic acid or the salt thereof coexist in the same food product, for example, combined as a compound food.

In some embodiments, the food is a dietary supplement or a food for special medical use.

In some embodiments, the weight ratio of the lysozyme to the glycyrrhizic acid or the salt thereof in the combination is 100 : 1 to 10 : 1. In some embodiments, the lysozyme and the glycyrrhizic acid or the salt thereof have a significant synergistic effect.

In some embodiments, the food is administrated by various manners, such as oral administration, nasogastric feeding, injection, inhalation etc.

In some embodiments, various release forms are applied for the food, such as normal release, delayed release, sustained release, and controlled release.

In some embodiments, the food further comprises an additive suitable for food, such as preservative, a colorant, a flavoring agent, a spice, an antistaling agent, an antioxidant, an emulsifier, a thickening agent, a carbohydrate, a fat, a vitamin, an amino acid, a trace element, or a protein.

In some embodiments, the daily dosage of the lysozyme is 0.5 g to 20 g.

In some embodiments, the food is administered during the non-sleeping time at a dosage frequency of once every 1 to 24 hours, such as once every 1 hour, every 2 hours, every 4 hours, every 6 hours, every 8 hours, every 12 hours, or every 24 hours.

### Beneficial Effects of the Invention:

The lysozyme or the combination of the lysozyme as provided by the present invention can effectively inhibit the cell damage caused by the COVID-19 coronavirus, inhibit the inflammatory response caused by the COVID-19 coronavirus, ameliorate airway obstruction and decreased expiratory volume caused by lung lesions, and improve the intestinal barrier function. It has a good therapeutical effect on high viral load, respiratory distress, impaired intestinal function, etc. in patients with the coronavirus pneumonia COVID-19. It can reduce both the 2019 novel coronavirus infection rate and the incidence rate of severe coronavirus pneumonia COVID-19. Moreover, in view of the extremely high safety of lysozyme, the lysozyme and the combination of the lysozyme also present great value for prevention against the coronavirus pneumonia COVID-19 in undiagnosed people at high risk of 2019 novel coronavirus infection.

### DETAILED DESCRIPTION OF EMBODIMENTS

The present invention is described in detail hereinafter with reference to particular embodiments. It is to be understood that the content of the particular embodiments is illustrative, rather than restrictive, that is, they do not limit the content of the present invention in any way.

### Definition:

The Chinese term "rongjunmei" refers to lysozyme. The lysozyme of the present invention may be either a lysozyme derived from an animal, a plant, or a microorganism, or a recombinant product of a natural lysozyme. For example, the lysozyme may be a hen egg white lysozyme, a human lysozyme, a recombinant human lysozyme, a bacteriophage lysozyme, etc. The lysozyme of the present invention includes a pharmaceutical salt thereof, such as a hydrochloride, chloride, sulfate and amino acid salt thereof. Lysozyme was first discovered by Fleming as an endogenous enzyme widely present in organisms. Lysozyme has been approved for use in food or medicine worldwide. In the United States lysozyme has been Generally Recognized As Safe(GRAS). Lysozyme has been permitted by WHO, many European countries, Japan, and China for use as food additives, and has been approved for use in medicine in China, Japan, Singapore, and other countries. At present, lysozyme is known to have a relatively strong antiviral ability against herpes viruses.

"COVID-19 coronavirus", namely 2019 Novel Coronavirus, is a virus discovered in 2019, and is a new virus belonging to the family of coronaviridae.

"Coronavirus pneumonia COVID-19" is a pneumonia caused by an infection with the COVID-19 coronavirus. The pathological characteristics, clinical manifestations, and diagnostic criteria of the coronavirus pneumonia COVID-19 are described in detail in the "Guidelines for the Diagnosis and Treatment of 2019 Novel Coronavirus (2019-nCoV) Infection" issued by the National Health Commission of the PRC. Many characteristics of the coronavirus pneumonia COVID-19 are obviously different from those of bacterial pneumonia, viral pneumonia, and atypical pneumonia(severe acute respiratory syndrome, SARS).

The present invention is further illustrated hereinafter with reference to **embodiments;** however, the **embodiments** do not limit the solutions and effects of the present invention.

### Example 1: Use of lysozyme in patients with coronavirus pneumonia COVID-19 and people at high risk of infection

During the coronavirus pneumonia COVID-19 epidemic in China in 2019, our company donated twice to Hubei Province medicines worth more than 2 million RMB, including antimicrobial medicines such as lysozyme lozenges and enteric-coated lysozyme tablets. We found that these medicines played a positive role in fighting against the COVID-19 epidemic.

Nine patients who were newly diagnosed with the coronavirus pneumonia COVID-19 were administered with the lysozyme lozenges and/or enteric-coated lysozyme tablets that we provided, at a dose ranging from 0.5 g to 2 g per day. The patients insisted on the administration almost every day, and as a result, only one patient progressed to a severe condition, while the other eight patients were all mildly ill and subsequently recovered. The rate of progression to severe condition was calculated to be 11.1%.

Three people who lived and traveled in high-risk areas during the epidemic insisted on taking lysozyme lozenges and/or enteric-coated lysozyme tablets every day, at a dose ranging from 1 g to 1.5 g per day, and they were not found to be infected with the 2019 novel coronavirus after the nucleic acid test when they returned to work, with the infection rate being 0%.

As reported in "The Epidemiological Characteristics of an Outbreak of 2019 Coronavirus Diseases (COVID-19) in China", the proportion of patients with severe and critical conditions, among patients with the coronavirus pneumonia COVID-19, was up to 18.5%, and after progression to severe and critical conditions, the mortality rate could be even closer to 50%. By comparison, we found that the administration of a lysozyme preparation could greatly reduce the severe disease rate and thereby reduce the mortality rate, and thus, it had a very positive effect on preventing and treating the COVID-19 epidemic. In addition, the lysozyme preparation also prevented people at high risk of 2019 novel coronavirus infection from being infected, which was of great value for controlling the spread and recurrence of the epidemic.

### Example 2: In vitro test of lysozyme against 2019 novel coronavirus

The effect of the lysozyme against 2019 novel coronavirus was studied using African green monkey kidney cells.

Drugs: Lysozyme and monoammonium glycyrrhizinate.

Cells: African green monkey kidney cells (VeroE6 cells).

Virus: COVID-19, with a titer of 100 TCID₅₀.

Culture: 100 µL of VeroE6 cells with a concentration of 2×10⁵ cells/mL were added to each well of a sterile 96-well culture plate, and cultured at 37°C for 24 hours. The culture plate was divided, by wells, into a blank control group, a virus control group, a low-dose lysozyme group, a medium-dose lysozyme group, a high-dose lysozyme group, a low-dose monoammonium glycyrrhizinate group, and a high-dose monoammonium glycyrrhizinate group, with 3 wells in each group. Except the blank control group, 100 µL/well of a 100 TCID₅₀ virus solution was added to each group, and after adsorption in a 5% CO₂ incubator at 37°C for 2 hours, the cell culture solution in the culture plate was discarded.

Addition of drugs: 100 µL/well of drug solutions were added to the wells of the groups according to the following dosages: the low-dose lysozyme group (500 µg/ml), the medium-dose lysozyme group (1000 µg/ml), the high-dose lysozyme group (2000 µg/ml), the low-dose monoammonium glycyrrhizinate group (1000 µg/ml), and the high-dose monoammonium glycyrrhizinate group (2000 µg/ml), and incubation was then performed at 37°C for 4 days.

Observation of cytopathic effects: After incubation was completed, the cytopathic effect (CPE) was observed under an optical microscope. The degree of the cytopathic effect found in the cells was recorded according to the following six levels: "-" which means no cytopathic effect, " ± " which means that the cytopathic effect is less than 10%, " + " which means that the cytopathic effect is about 25%, " ++ " which means that the cytopathic effect is about 50%, " +++ " which means that the cytopathic effect is about 75%, and " ++++ " which means that the cytopathic effect is no less than 75%. The inhibition rate of the drug in each group on the cytopathic effect caused by the virus was calculated. The higher the inhibition rate, the better the effect.

Results: The main results of the test were listed in Table 1. The test results showed that the lysozyme significantly inhibited the cytopathic effect caused by the COVID-19 virus. The glycyrrhetate had a relatively weak inhibition effect on the COVID-19 virus.

**Table 1: Inhibition rate of lysozyme on cytopathic effect caused by COVID-19 virus**

| Drug (concentration) | Inhibition rate (%) |
|---|---|
| Lysozyme (500 µg/ml) | 15.00 ± 8.66 |
| Lysozyme (1000 µg/ml) | 46.67 ± 15.28 |
| Lysozyme (2000 µg/ml) | 78.33 ± 2.89 |
| Monoammonium glycyrrhizinate (1000 µg/ml) | 23.35 ± 5.43 |
| Monoammonium glycyrrhizinate (2000 µg/ml) | 29.42 ± 7.16 |

### Example 3: In vitro test of combinations comprising the lysozyme against 2019 novel coronavirus and inflammation caused by 2019 novel coronavirus

The inhibition effect of combinations comprising the lysozyme on cytopathic effect and inflammation caused by the COVID-19 virus was studied.

The drugs, cells, viruses etc. were all the same as those in Example 2.

The grouping in a culture plate and the dosages of the drugs were as follows: a blank control group, a virus control group, a lysozyme group (350 µg/ml), lysozyme and monoammonium glycyrrhizinate group I (100 µg/ml + 1 µg/ml), lysozyme and monoammonium glycyrrhizinate group II (100 µg/ml + 5 µg/ml), lysozyme and monoammonium glycyrrhizinate group III (100 µg/ml + 10 µg/ml), and lysozyme and monoammonium glycyrrhizinate group IV (100 µg/ml + 50 µg/ml).

The method for observing the cytopathic effect was the same as that in Example 2, and the test results were shown in Table 2. In addition, cells without any cytopathic effect were collected, RNA was extracted, and the relative expression levels of inflammatory factors such as TNF-α and IL-6 were determined by means of a quantitative PCR method. The test results were shown in Table 3.

**Table 2: Inhibition effects of combinations comprising lysozyme on cytopathic effect caused by COVID-19 virus**

| Drug (concentration) | Inhibition rate (%) |
|---|---|
| Lysozyme (350 µg/ml) | 6.67 ± 1.56 |
| Lysozyme + monoammonium glycyrrhizinate (100 µg/ml + 1 µg/ml) | 38.20 ± 8.11## |
| Lysozyme + monoammonium glycyrrhizinate (100 µg/ml + 5 µg/ml) | 49.32 ± 9.45## |
| Lysozyme + monoammonium glycyrrhizinate (100 µg/ml + 10 µg/ml) | 23.68 ± 4.31## |
| Lysozyme + monoammonium glycyrrhizinate (100 µg/ml + 50 µg/ml) | 8.20 ± 2.27 |

| | |
|---|---|
| Note: comparing the drug combination groups with the lysozyme group, p < 0.05 (#), and p < 0.01 (##). | |

**Table 3: Effects of combinations comprising lysozyme on cell inflammatory factors expression induced by COVID-19 virus**

| Group | Relative expression quantity of TNF-α mRNA | Relative expression quantity of IL-6 mRNA |
|---|---|---|
| Blank control group | 1.01 ± 0.06 | 1.08 ± 0.07 |
| Virus control group | 3.61 ± 0.29 | 7.68 ± 0.50 |
| Lysozyme group (350 µg/ml) | 3.12 ± 0.26* | 6.78 ± 0.30** |
| Lysozyme + monoammonium glycyrrhizinate | 2.25 ± 0.36**## | 4.30 ± 0.21**## |
| group I (100 µg/ml + 1 µg/ml) | | |
| Lysozyme + monoammonium glycyrrhizinate | 2.13 ± 0.14**## | 3.47 ± 0.25**## |
| group II (100 µg/ml + 5 µg/ml) | | |
| Lysozyme + monoammonium glycyrrhizinate | 2.65 ± 0.26**# | 5.74 ± 0.31**## |
| group III (100 µg/ml + 10 µg/ml) | | |
| Lysozyme + monoammonium glycyrrhizinate | 3.15 ± 0.10 | 6.45 ± 0.43** |
| group IV (100 µg/ml + 50 µg/ml) | | |

| | | |
|---|---|---|
| Note: comparing the drug groups with the virus control group, p < 0.05 (*) and p < 0.01 (**). Comparing the drug combination groups with the lysozyme group, p < 0.05 (#) and p < 0.01 (##). | | |

It could be seen from Tables 2 and 3 that the lysozyme and the drug combinations comprising the lysozyme had a better inhibition effect on the cytopathic effect caused by the COVID-19 virus, and could significantly reduce the increase of the cell inflammatory factors, as caused by the COVID-19 virus. Compared with the lysozyme alone, effects of drug combinations were obviously better, which manifested in a significant increase in the inhibition rate, a significant reduction in the levels of inflammatory factors, and a substantial reduction in the dosage of the drugs. It was suggested that there was a strong synergistic effect between glycyrrhetate and lysozyme, and in particular, when the dosage of the glycyrrhetate was relatively low, the synergistic effect was better.

### Example 4: Effects of lysozyme and the lysozyme combinations on airway resistance and lung function of guinea pigs which had inhaled smoke

The smoke-induced model was a classic animal model for chronic obstructive pulmonary disease. After inhaling smoke for a long period of time, animals could suffer from increased mucus secretion, airway obstruction, lung function decline, etc. The effect of the lysozyme on airway obstruction was evaluated by means of the model establishment method in the present experiment.

### 1. Test drugs

Lysozyme, monoammonium glycyrrhizinate, and monopotassium glycyrrhizinate.

### 2. Animals and grouping

Male Hartley guinea pigs, weighing 400 to 500 g, were randomly divided into a blank group, a model group, a lysozyme oral administration group (200 mg/kg), a lysozyme inhalation group (2 mg/kg), lysozyme + monoammonium glycyrrhizinate inhalation group (2 mg/kg + 0.1 mg/kg), lysozyme + monopotassium glycyrrhizinate oral administration group I (200 mg/kg + 20 mg/kg), and lysozyme + monopotassium glycyrrhizinate oral administration group II (200 mg/kg + 40 mg/kg), with eight animals in each group.

### 3. Model establishment

The animals in each group except the blank group were exposed to cigarette smoke by using a smoke exposure system for oro-nasal inhalation only, such that the animals inhaled smoke of five cigarettes within 40 minutes per day, for 5 exposure days per week, for 12 consecutive weeks.

### 4. Preparation and administration of drugs

The lysozyme and monoammonium glycyrrhizinate used for inhalation administration were respectively crushed into powders with a particle size of less than 10 µm. The lysozyme and monopotassium glycyrrhizinate used for oral administration were respectively dissolved in normal saline.

The animals in each group except the blank group and the model group were administered once a day for 4 consecutive weeks from the 8^{th} week after the model establishment began. The inhalation administration groups were administered by means of an oro-nasal inhalation exposure system, while the oral administration groups were administered intragastrically. Animals in the blank group and the model group were administered intragastrically with normal saline per day.

### 5. Test

Airway resistance measurement and 100 ms(millisecond) expiratory volume measurement were performed after administration.

The animals were placed in a double chamber plethysmography system, and after the animals were left in a tranquil state for 10 minutes, the specific airway resistance (sRaw) was measured. The animals were anesthetized by intramuscular injection of ketamine, then a catheter was then inserted into the trachea, and the forced expiratory volume in 100 ms (FEV 100) of the animals was measured through a lung function testing system.

### 6. Test results and evaluation

The results of the airway resistance measurement and the 100 ms expiratory volume measurement of the animals were as shown in Figure 4.

**Table 4: Effects of lysozyme and lysozyme combinations on airway resistance and 100 ms expiratory volume of animals**

| Group | Airway resistance (cmH2O•s) | 100 ms expiratory volume (mL) |
|---|---|---|
| Blank group | 1.43 ± 0.24 | 11.86 ±1.24 |
| Model group | 4.72 ± 0.38 | 4.18 ± 1.26 |
| Lysozyme oral administration group | 3.79 ± 0.30** | 5.79 ± 2.03 |
| Lysozyme inhalation group | 3.10 ± 0.14** | 7.68 ± 1.36** |
| Lysozyme + monoammonium glycyrrhizinate inhalation group | 2.52 ± 0.22**&& | 9.04 ± 0.84** |
| Lysozyme + monopotassium glycyrrhizinate oral administration group I | 2.78 ± 0.17**## | 8.05 ± 1.56**# |
| Lysozyme + monopotassium glycyrrhizinate oral administration group II | 3.47 ± 0.28** | 5.89 ± 1.01 |

| | | |
|---|---|---|
| Note: comparing the administration groups with the model group, p < 0.05 (*) and p < 0.01 (**). Comparing the combination oral administration groups with the lysozyme oral administration group, p < 0.05 (#) and p < 0.01 (##). Comparing the combination inhalation groups with the lysozyme inhalation group, p < 0.05(&) and p < 0.01(&&). | | |

In the test, long-term inhalation of smoke caused a significant increase in the lung airway resistance and a significant decline in the lung function (reduced expiratory volume) in the animals. It could be seen from the test results that these administration groups reduced the airway resistance and increased the expiratory volume in the animals to varying degrees. By comparing the drug combination groups with the lysozyme-only groups, it was found that the glycyrrhizic acid-based substance enhanced the effect of the lysozyme, and in particular, when the dosage of the glycyrrhizic acid-based substance was relatively small, the synergistic effect was significant.

### Example 5: Protective effects of lysozyme and the lysozyme combinations against lipopolysaccharide-induced intestinal barrier dysfunction in mice

Lipopolysaccharides could cause impaired intestinal function in animals. The protective effect of lysozyme and lysozyme combinations against lipopolysaccharide-induced intestinal barrier dysfunction in mice were studied in the present experiment.

Test drugs: lysozyme, monoammonium glycyrrhizinate, and dipotassium glycyrrhizinate.

Test animals: clean-grade male C57BL/6 mice, weighing 20 to 25 g.

Animal grouping and administration: The animals were randomly divided into a blank group, a model group, a lysozyme group (100 mg/kg), lysozyme + monoammonium glycyrrhizinate group A (100 mg/kg + 2 mg/kg), lysozyme + monoammonium glycyrrhizinate group B (100 mg/kg + 10 mg/kg), lysozyme + monoammonium glycyrrhizinate group C (100 mg/kg + 20 mg/kg), and a lysozyme + dipotassium glycyrrhizinate group (100 mg/kg + 2 mg/kg), with eight animals in each group. Each drug group was administered intragastrically by each dose of drugs every day, while the blank group and the model group were administered with normal saline. The administration was performed for 30 consecutive days.

Model establishment: After administration, the animals of each group except the blank group were intraperitoneally injected with 15 mg/kg of lipopolysaccharides, and the blank group was injected with normal saline. The animals were fasted for 12 hours after injection.

Determination of intestinal permeability: The animals of each group were administered intragastrically with 600 mg/kg of FITC-glucan (dissolved in a phosphate buffer), and after 4 hours, blood was taken from eyeballs. The content of the FITC-glucan in the serum was measured by means of fluorescence spectrometry (with an excitation wavelength of 480 nm and an emission wavelength of 520 nm), and the results were shown in Table 5.

Determination of expression of tight junction proteins in intestinal tissue: After the animals were sacrificed, jejunum tissue samples were taken for RNA extraction, the relative expression levels of occludin protein and Claudin-1 protein in the tissues were detected by quantitative PCR and the results were shown in Table 6.

**Table 5: Effects of lysozyme and lysozyme combinations on intestinal permeability**

| Group | Content of FITC-glucan (µg/ml) |
|---|---|
| Blank group | 9.40 ± 1.45 |
| Model group | 35.06 ± 5.75 |
| Lysozyme group (100 mg/kg) | 27.53 ± 3.21** |
| Lysozyme + monoammonium glycyrrhizinate group A (100 mg/kg + 2 mg/kg) | 15.31 ± 2.08**## |
| Lysozyme + monoammonium glycyrrhizinate group B (100 mg/kg + 10 mg/kg) | 20.25 ± 2.44**## |
| Lysozyme + monoammonium glycyrrhizinate group C (100 mg/kg + 20 mg/kg) | 23.89 ± 2.52** |
| Lysozyme + dipotassium glycyrrhizinate group (100 mg/kg + 2 mg/kg) | 13.17 ± 1.14**## |

| | |
|---|---|
| Note: comparing the drug groups with the model group, p < 0.05 (*) and p < 0.01 (**). Comparing the drug combination groups with the lysozyme group, p < 0.05 (#) and p < 0.01 (##). | |

**Table 6: Effects of lysozyme and lysozyme combinations on expression of tight junction proteins in intestinal tissue**

| Group | Relative expression quantity of Occludin mRNA | Relative expression quantity of Claudin-1 mRNA |
|---|---|---|
| Blank group | 1.04 ± 0.12 | 1.07 ± 0.11 |
| Model group | 0.54 ± 0.09 | 0.73 ± 0.13 |
| Lysozyme group (100 mg/kg) | 0.69 ± 0.07** | 0.79 ± 0.16 |
| Lysozyme + monoammonium glycyrrhizinate | 1.22 ± 0.24**## | 1.30 ± 0.19**## |
| group A (100 mg/kg + 2 mg/kg) | | |
| Lysozyme + monoammonium glycyrrhizinate | 0.93 ± 0.16**## | 1.11 ± 0.15**## |
| group B (100 mg/kg + 10 mg/kg) | | |
| Lysozyme + monoammonium glycyrrhizinate | 0.67 ± 0.10* | 0.83 ± 0.17 |
| group C (100 mg/kg + 20 mg/kg) | | |
| Lysozyme + dipotassium glycyrrhizinate group (100 mg/kg + 2 mg/kg) | 1.07 ± 0.07**## | 1.00 ± 0.16**# |

| | | |
|---|---|---|
| Note: comparing the drug groups with the model group, p < 0.05 (*) and p < 0.01 (**). Comparing the drug combination groups with the lysozyme group, p < 0.05 (#) and p < 0.01 (##). | | |

The protective effects of the lysozyme and the lysozyme combinations against lipopolysaccharide-induced intestinal barrier dysfunction in mice were studied in the present experiment. After lipopolysaccharide injection, it was found that the content of the glucan in serum in the model group was significantly increased as compared with that in the blank group, indicating that a large amount of the glucan entered the blood through the intestine, that is, the intestinal permeability was increased; in addition, the expression of the two tight junction proteins in the intestine was decreased in the model group, further indicating that the intestinal barrier function was impaired. It was found through the present experiment that the lysozyme and the lysozyme combinations could significantly reduce the increase in the intestinal permeability induced by the lipopolysaccharide, significantly increase the expression of the tight junction proteins in the intestinal tissues, and had an obvious protective effect against intestinal barrier dysfunction. It was also found that the effect of the drug combination groups was obviously better than that of the lysozyme used alone.

In conjunction with a plurality of examples above, it is obvious that the lysozyme and the lysozyme combinations can inhibit cell damage caused by the 2019 novel coronavirus, inhibit the inflammatory factors expression induced by the 2019 novel coronavirus, ameliorate lung airway obstruction, increase the expiratory volume, ameliorate intestinal barrier dysfunction, and can reduce the 2019 novel coronavirus infection rate and the rate of progression to severe coronavirus pneumonia COVID-19. The coronavirus pneumonia COVID-19 is still a new thing in the medical field. According to the current clinical knowledge, it has been shown that the patients have a high viral load in the early stage and obvious respiratory distress symptoms in the middle and late stages, and the intestinal barrier dysfunction leads to persistent and secondary infections, difficult rehabilitation, and increased infectivity in the patients, as well as a high mortality rate in severe patients, etc. Therefore, the lysozyme and the lysozyme combinations of the present invention are expected to prevent and treat the coronavirus pneumonia COVID-19 in various ways, and due to the good safety thereof, the dosage can be increased. The lysozyme and the lysozyme combinations are expected to become a better choice for preventing or treating the coronavirus pneumoniaCOVID-19.

## Claims

1. A medicament for use in preventing or treating the coronavirus pneumonia COVID-19, wherein the medicament comprises lysozyme and glycyrrhizic acid or a salt thereof, wherein the weight ratio of the lysozyme to the glycyrrhizic acid or the salt thereof in the medicament is 100:1 to 10:1.

2. The medicament for use according to claim 1, further comprising an additional ingredient useful for preventing or treating the coronavirus pneumonia COVID-19, wherein said additional ingredient is at least one selected from the group consisting of interferon, oseltamivir or salts thereof, lopinavir, ritonavir, favipiravir, ribavirin, remdesivir, chloroquine or salts thereof, hydroxychloroquine or salts thereof, arbidol or salts thereof, interleukin inhibitors, tumor necrosis factor inhibitors, janus kinase inhibitors, glucocorticoid, protease inhibitors, or intestinal microecological regulators; and
the protease inhibitors are selected from ulinastatin, sivelestat, nafamostat, or tranexamic acid; the interleukin inhibitors are selected from tocilizumab; the tumor necrosis factor inhibitors are selected from adalimumab, infliximab, or etanercept; the janus kinase inhibitors are selected from tofacitinib or baricitinib; the intestinal microecological regulators are selected from prebiotics or probiotics.

3. The medicament for use according to claim 1, wherein dosage form of the medicament is an oral dosage form, an injection dosage form, or an inhalation dosage form.

4. The medicament for use according to claim 1, wherein the release form of the medicament is normal release, delayed release, sustained release, or controlled release.

5. A combination for use in preventing or treating the coronavirus pneumonia COVID-19, wherein the combination comprises an effective amount of lysozyme and glycyrrhizic acid or a salt thereof, wherein the weight ratio of the lysozyme to the glycyrrhizic acid or the salt thereof in the medicament is 100:1-10:1.

6. The combination for use according to claim 5, wherein the combination comprising lysozyme is prepared into a food.

7. The combination for use according to claim 6, wherein the lysozyme and the glycyrrhizic acid or the salt thereof in the combination exist separately in different pharmaceutical products or food products, or the lysozyme and the glycyrrhizic acid or the salt thereof coexist in the same pharmaceutical product or food product.

8. The combination for use according to claim 6, wherein the combination further comprises an additional ingredient useful for preventing or treating the coronavirus pneumonia COVID-19, and the additional ingredient useful for preventing or treating the coronavirus pneumonia COVID-19 is at least one selected from the group consisting of interferon, oseltamivir or salts thereof, lopinavir, ritonavir, favipiravir, ribavirin, remdesivir, chloroquine or salts thereof, hydroxychloroquine or salts thereof, arbidol or salts thereof, interleukin inhibitors, tumor necrosis factor inhibitors, janus kinase inhibitors, glucocorticoid, protease inhibitors, or intestinal microecological regulators; wherein the protease inhibitors are selected from ulinastatin, sivelestat, nafamostat, or tranexamic acid; the interleukin inhibitors are selected from tocilizumab; the tumor necrosis factor inhibitors are selected from adalimumab, infliximab, or etanercept; the janus kinase inhibitors are selected from tofacitinib or baricitinib; the intestinal microecological regulators are selected from prebiotics or probiotics.

9. The combination for use according to claim 8, wherein the lysozyme and the glycyrrhizic acid or the salt thereof in the combination exist separately in different pharmaceutical products, or the lysozyme and the glycyrrhizic acid or the salt thereof coexist in the same pharmaceutical product.

10. The combination for use according to any one of claims 5 to 9, wherein the daily dosage of the lysozyme is 0.5 g to 20 g.

11. The combination for use according to any one of claims 5 to 10, wherein the subject to be administered with said lysozyme or combination comprising lysozyme is tested positive for COVID-19 coronavirus nucleic acid, or is clinically diagnosed with the coronavirus pneumonia COVID-19.

12. The combination for use according to claim 11, wherein said subject suffers from increased airway resistance, decreased expiratory volume, and/or impaired intestinal barrier function.

## Patentansprüche

1. Medikament zur Verwendung bei der Vorbeugung oder Behandlung der Coronavirus-Pneumonie COVID-19, wobei das Medikament Lysozym und Glycyrrhizinsäure oder ein Salz davon umfasst, wobei das Gewichtsverhältnis von Lysozym zu Glycyrrhizinsäure oder dem Salz davon in dem Medikament 100:1 bis 10:1 beträgt.

2. Medikament zur Verwendung gemäß Anspruch 1, ferner umfassend einen zusätzlichen Bestandteil, der zur Vorbeugung oder Behandlung der Coronavirus-Pneumonie COVID-19 nützlich ist, wobei der zusätzliche Bestandteil mindestens einer ist, ausgewählt aus der Gruppe bestehend aus Interferon, Oseltamivir oder Salzen davon, Lopinavir, Ritonavir, Favipiravir, Ribavirin, Remdesivir, Chloroquin oder Salzen davon, Hydroxychloroquin oder Salzen davon, Arbidol oder Salzen davon, Interleukin-Inhibitoren, Tumornekrosefaktor-Inhibitoren, Januskinase-Inhibitoren, Glucocorticoid, Protease-Inhibitoren oder intestinalen mikroökologischen Regulatoren; und
Proteaseinhibitoren, die ausgewählt sind aus Ulinastatin, Sivelestat, Nafamostat oder Tranexamsäure; Interleukininhibitoren, die ausgewählt sind aus Tocilizumab; Tumornekrosefaktorinhibitoren, die ausgewählt sind aus Adalimumab, Infliximab oder Etanercept; Januskinaseinhibitoren, die ausgewählt sind aus Tofacitinib oder Baricitinib; intestinalen mikroökologischen Regulatoren, die ausgewählt sind aus Präbiotika oder Probiotika.

3. Medikament zur Verwendung nach Anspruch 1, wobei die Dosierungsform des Medikaments eine orale Dosierungsform, eine Injektionsdosierungsform oder eine Inhalationsdosierungsform ist.

4. Medikament zur Verwendung nach Anspruch 1, wobei die Freisetzungsform des Medikaments normale Freisetzung, verzögerte Freisetzung, verzögerte Freisetzung oder kontrollierte Freisetzung ist.

5. Kombination zur Verwendung bei der Vorbeugung oder Behandlung der Coronavirus-Pneumonie COVID-19, wobei die Kombination eine wirksame Menge an Lysozym und Glycyrrhizinsäure oder einem Salz davon umfasst, wobei das Gewichtsverhältnis von Lysozym zu Glycyrrhizinsäure oder dem Salz davon in dem Medikament 100:1-10:1 beträgt.

6. Kombination zur Verwendung nach Anspruch 5, wobei die Lysozym enthaltende Kombination zu einem Lebensmittel verarbeitet wird.

7. Kombination zur Verwendung nach Anspruch 6, wobei das Lysozym und die Glycyrrhizinsäure oder das Salz davon in der Kombination getrennt in verschiedenen pharmazeutischen Produkten oder Lebensmitteln vorhanden sind oder das Lysozym und die Glycyrrhizinsäure oder das Salz davon in demselben pharmazeutischen Produkt oder Lebensmittel koexistieren.

8. Kombination zur Verwendung gemäß Anspruch 6, wobei die Kombination ferner einen zusätzlichen Bestandteil umfasst, der zur Vorbeugung oder Behandlung der Coronavirus-Pneumonie COVID-19 nützlich ist, und der zusätzliche Bestandteil, der zur Vorbeugung oder Behandlung der Coronavirus-Pneumonie COVID-19 nützlich ist, mindestens einer ist, ausgewählt aus der Gruppe bestehend aus Interferon, Oseltamivir oder Salzen davon, Lopinavir, Ritonavir, Favipiravir, Ribavirin, Remdesivir, Chloroquin oder Salzen davon, Hydroxychloroquin oder Salzen davon, Arbidol oder Salzen davon, Interleukin-Inhibitoren, Tumornekrosefaktor-Inhibitoren, Januskinase-Inhibitoren, Glucocorticoid, Protease-Inhibitoren oder mikroökologischen Regulatoren des Darms; wobei die Proteaseinhibitoren ausgewählt sind aus Ulinastatin, Sivelestat, Nafamostat oder Tranexamsäure; die Interleukin-inhibitoren ausgewählt sind aus Tocilizumab; die Tumornekrosefaktorinhibitoren ausgewählt sind aus Adalimumab, Infliximab oder Etanercept; die Januskinaseinhibitoren ausgewählt sind aus Tofacitinib oder Baricitinib; die mikroökologischen Darmregulatoren ausgewählt sind aus Präbiotika oder Probiotika.

9. Kombination zur Verwendung nach Anspruch 8, wobei das Lysozym und die Glycyrrhizinsäure oder das Salz davon in der Kombination getrennt in verschiedenen pharmazeutischen Produkten vorliegen oder das Lysozym und die Glycyrrhizinsäure oder das Salz davon in demselben pharmazeutischen Produkt koexistieren.

10. Kombination zur Verwendung nach einem der Ansprüche 5 bis 9, wobei die Tagesdosis des Lysozyms 0,5 g bis 20 g beträgt.

11. Kombination zur Verwendung nach einem der Ansprüche 5 bis 10, wobei das mit dem Lysozym oder der Lysozym enthaltenden Kombination zu verabreichende Subjekt positiv auf COVID-19-Coronavirus-Nukleinsäure getestet ist oder klinisch mit der Coronavirus-Pneumonie COVID-19 diagnostiziert wurde.

12. Kombination zur Verwendung gemäß Anspruch 11, wobei das Subjekt unter einem erhöhten Atemwegswiderstand, einem verringerten Exspirationsvolumen und/oder einer beeinträchtigten Darmbarrierefunktion leidet.

## Revendications

1. Médicament utilisé pour prévenir ou traiter la pneumonie à coronavirus COVID-19, dans lequel le médicament comprend du lysozyme et de l'acide glycyrrhizique ou un de ses sels, le rapport pondéral entre le lysozyme et l'acide glycyrrhizique ou son sel dans le médicament étant compris entre 100:1 et 10:1.

2. Médicament à utiliser selon la revendication 1, comprenant en outre un ingrédient supplémentaire utile pour prévenir ou traiter la pneumonie à coronavirus COVID-19, dans lequel ledit ingrédient supplémentaire est au moins un ingrédient choisi dans le groupe constitué de l'interféron, de l'oseltamivir ou de ses sels, du lopinavir, ritonavir, favipiravir, ribavirine, remdesivir, chloroquine ou ses sels, hydroxychloroquine ou ses sels, arbidol ou ses sels, inhibiteurs de l'interleukine, inhibiteurs du facteur de nécrose tumorale, inhibiteurs de la janus kinase, glucocorticoïdes, inhibiteurs de la protéase ou régulateurs microécologiques intestinaux ; et
les inhibiteurs de protéase sont choisis parmi l'ulinastatine, le sivelestat, le nafamostat ou l'acide tranexamique ; les inhibiteurs d'interleukines sont choisis parmi le tocilizumab ; les inhibiteurs du facteur de nécrose tumorale sont choisis parmi l'adalimumab, l'infliximab ou l'étanercept ; les inhibiteurs de la janus kinase sont choisis parmi le tofacitinib ou le baricitinib ; les régulateurs microécologiques intestinaux sont choisis parmi les prébiotiques ou les probiotiques.

3. Médicament à utiliser selon la revendication 1, dans lequel la forme posologique du médicament est une forme posologique orale, une forme posologique injectable ou une forme posologique par inhalation.

4. Médicament à utiliser selon la revendication 1, dans lequel la forme de libération du médicament est à libération normale, à libération retardée, à libération prolongée ou à libération contrôlée.

5. Combinaison utilisée pour prévenir ou traiter la pneumonie à coronavirus COVID-19, dans laquelle la combinaison comprend une quantité efficace de lysozyme et d'acide glycyrrhizique ou d'un sel de celui-ci, le rapport pondéral entre le lysozyme et l'acide glycyrrhizique ou le sel de celui-ci dans le médicament étant de 100:1-10:1.

6. Combinaison d'utilisation selon la revendication 5, dans laquelle la combinaison comprenant le lysozyme est préparée dans un aliment.

7. Combinaison utilisable selon la revendication 6, dans laquelle le lysozyme et l'acide glycyrrhizique ou son sel dans la combinaison existent séparément dans différents produits pharmaceutiques ou produits alimentaires, ou le lysozyme et l'acide glycyrrhizique ou son sel coexistent dans le même produit pharmaceutique ou produit alimentaire.

8. Combinaison utilisable selon la revendication 6, dans laquelle la combinaison comprend en outre un ingrédient supplémentaire utile pour prévenir ou traiter la pneumonie à coronavirus COVID-19, et l'ingrédient supplémentaire utile pour prévenir ou traiter la pneumonie à coronavirus COVID-19 est au moins un ingrédient choisi dans le groupe constitué de l'interféron, oseltamivir ou ses sels, lopinavir, ritonavir, favipiravir, ribavirine, remdesivir, chloroquine ou ses sels, hydroxychloroquine ou ses sels, arbidol ou ses sels, inhibiteurs de l'interleukine, inhibiteurs du facteur de nécrose tumorale, inhibiteurs de la janus kinase, glucocorticoïdes, inhibiteurs de la protéase ou régulateurs microécologiques intestinaux ; dans lequel les inhibiteurs de protéase sont choisis parmi l'ulinastatine, le sivelestat, le nafamostat ou l'acide tranexamique ; les inhibiteurs d'interleukines sont choisis parmi le tocilizumab ; les inhibiteurs du facteur de nécrose tumorale sont choisis parmi l'adalimumab, l'infliximab ou l'étanercept ; les inhibiteurs de janus kinase sont choisis parmi le tofacitinib ou le baricitinib ; les régulateurs microécologiques intestinaux sont choisis parmi les prébiotiques ou les probiotiques.

9. Combinaison d'utilisation selon la revendication 8, dans laquelle le lysozyme et l'acide glycyrrhizique ou son sel dans la combinaison existent séparément dans différents produits pharmaceutiques, ou le lysozyme et l'acide glycyrrhizique ou son sel coexistent dans le même produit pharmaceutique.

10. L'association à utiliser selon l'une des revendications 5 à 9, dans laquelle la dose quotidienne de lysozyme est comprise entre 0,5 g et 20 g.

11. Combinaison utilisable selon l'une des revendications 5 à 10, dans laquelle le sujet à qui l'on doit administrer ce lysozyme ou cette combinaison comprenant du lysozyme est testé positif pour l'acide nucléique du coronavirus COVID-19, ou est cliniquement diagnostiqué avec le coronavirus de la pneumonie COVID-19.

12. Combinaison d'utilisation selon la revendication 11, dans laquelle le sujet souffre d'une résistance accrue des voies respiratoires, d'une diminution du volume expiratoire et/ou d'une altération de la fonction de la barrière intestinale.
